Europäisches Patentamt

European Patent Office

Office européen des brevets

(19)

(11) **EP 1 009 368 B1**

(12) **EUROPÄISCHE PATENTSCHRIFT**

(45) Veröffentlichungstag und Bekanntmachung des Hinweises auf die Patenterteilung:
**22.12.2004   Patentblatt 2004/52**

(51) Int Cl.⁷: **A61K 7/06**

(21) Anmeldenummer: **98954255.0**

(22) Anmeldetag: **05.09.1998**

(86) Internationale Anmeldenummer:
**PCT/EP1998/005631**

(87) Internationale Veröffentlichungsnummer:
**WO 1999/013821 (25.03.1999 Gazette 1999/12)**

(54) **SPLISSREGENERIERENDE HAARBEHANDLUNGSMITTEL**

HAIR CONDITIONERS FOR TREATING SPLIT ENDS

AGENTS DE SOINS CAPILLAIRES POUR TRAITER LES FOURCHES

(84) Benannte Vertragsstaaten:
**AT BE CH DE DK ES FI FR GB GR IT LI NL PT SE**
Benannte Erstreckungsstaaten:
**SI**

(30) Priorität: **13.09.1997  DE 19740285**

(43) Veröffentlichungstag der Anmeldung:
**21.06.2000   Patentblatt 2000/25**

(73) Patentinhaber: **Henkel Kommanditgesellschaft auf Aktien**
**40589 Düsseldorf-Holthausen (DE)**

(72) Erfinder:
• **BERNECKER, Ullrich**
**D-52393 Hürtgenwald (DE)**

• **BROCKMANN, Claudia**
**D-40627 Düsseldorf (DE)**
• **HOLLENBERG, Detlef**
**D-40699 Erkrath (DE)**

(56) Entgegenhaltungen:
**EP-A- 0 287 876        WO-A-96/14824**
**WO-A-96/32926        DE-A- 19 613 567**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft Haarbehandlungsmittel, die in Form einer Öl-in-Wasser-Emulsion vorliegen und eine besonders wirksame Kombination von lipidlöslichen und wasserlöslichen haarsplißreduzierenden Wirkstoffen enthalten.

Haarspliß ist eine Erscheinung, die sich in einem Poröswerden des Haarschaftes und einer Aufspaltung des Haars von der Haarspitze her manifestiert. Haarspliß wird u.a. durch starke mechanische Beanspruchung des Haars, z.B. durch häufiges Bürsten, Toupieren oder Kämmen gegen hohen Kämmwiderstand verursacht. Ursache für hohen Kämmwiderstand bei trockenem Haar kann Schädigung der Haaroberfläche, statische Aufladung oder Klebrigkeit infolge von Rückständen von Haarsprays sein. Die Gefahr von Haarspliß wird auch durch eine Schwächung der Haarstruktur erhöht, die durch häufige oder unsachgemäße Anwendung chemischer Behandlungsverfahren, z.B. beim Dauerwellen oder Färben, verursacht sein kann.

Es hat daher nicht an Versuchen gefehlt, durch Haarspliß geschädigtes Haar mit Hilfe geeigneter Zubereitungen zu regenerieren, das heißt, den weiteren Fortschritt des Haarsplisses aufzuhalten und das gesplißte Haar zu sanieren.

In DE 195 14 268 sind kosmetische Mittel beschrieben, die Esterpolyole enthalten, wobei diese Stoffe bei Anwendung auf dem Haar dessen Reißfestigkeit erhöhen können. Über eine Regenerierung von gesplißten Haaren ist dieser Druckschrift nichts zu entnehmen.

Aus DE 37 11 841 A1 sind haarregenerierende Zubereitungen bekannt, die als wasserlösliche Wirkstoffe zur Verringerung des Haarsplisses z.B. Panthenol oder Glucose, gegebenenfalls in Kombination mit Polyvinylpyrrolidon enthalten.

Aus DE 44 40 315 A1 war bekannt, daß Kombinationen von Mono- oder Oligosacchariden und deren Derivaten mit kationisch derivatisiertem Panthenol eine Regeneration gesplißter Haare bewirken können.

Die zuletzt genannten Zusammensetzungen verringern zwar die Spließrate geschädigter Haare, die Wirkung ist jedoch bei Produkten, die nach der Anwendung wieder vom Haar abgespült werden (den sogenannten "rinse-off"-Produkten) wie z.B. Shampoos und Haarspülungen noch nicht in jeder Hinsicht befriedigend.

Es bestand daher die Aufgabe, Haarbehandlungsmittel in Bezug auf den spließheilenden Effekt deutlich zu verbessern. Diese Aufgabe wurde durch die erfindungsgemäßen, spließregenerierenden Haarbehandlungsmittel in besonders hohem Maße erfüllt.

Gegenstand der Erfindung sind Zubereitungen zur Behandlung von Haaren in Form einer Öl-in-Wasser-Emulsion, dadurch gekennzeichnet, daß als spließvermindemde Wirkstoffe

0,1 - 10 Gew.-%    eines lipidlöslichen Esteralkohols oder Esterpolyols, erhältlich durch Umsetzung eines Epoxyfettsäureesters mit einem ein- oder

mehrwertigen Alkohol mit 1 - 6 C-Atomen als Komponente der Ölphase und

0,1 - 3 Gew.-%    einer bekannten, haarspließvermindernden, wasserlöslichen Verbindung aus der Gruppe Panthenol, Nicotinsäureamid, Zucker, Polyvinylpyrrolidon oder Gemischen davon als Komponente der wäßrigen Phase

enthalten sind.

Als Zubereitungen im Sinne der Erfindung sind sowohl "leave-on"-Produkte wie z.B. Haarfestiger, Frisierhilfsmittel und Haarkurprodukte, wie auch solche Produkte zu verstehen, die nach der Anwendung wieder aus dem Haar ausgespült werden, wie z.B. Shampoos, Haarnachspülmittel, Dauerwell-Fixiermittel oder z.B. Haarfärbe-mittel.

Wichtig ist lediglich, daß die Zubereitungen eine emulgierte Ölphase enthalten, also in Form einer Öl-in-Wasser-Emulsion oder -Mikroemulsion vorliegen.

Die lipidlöslichen Esteralkohole oder Esterpolyole sind literaturbekannte und im Handel erhältliche Produkte. Als lipidlöslich sind sie dann anzusehen, wenn sich 5 Gew.-% dieser Produkte in Cetylalkohol bei 80° C klar auflösen.

Die erfindungsgemäß geeigneten Esteralkohole oder Esterpolyole sind erhältlich durch Umsetzung eines Epoxyfettsäureesters mit Wasser oder ein- oder mehrwertigen Alkoholen mit 1 - 10 C-Atomen unter Öffnung des Epoxidrings und Ausbildung einer vizinalen Dihydroxyethyl- oder Hydroxy-alkoxyethylgruppe. Der Epoxyfettsäureester kann dabei auch ein Epoxidationsprodukt aus einem technischen Fettsäureester mit Anteilen gesättigter Fettsäuren sein. Der Epoxidsauerstoffgehalt sollte aber wenigstens 3 Gew.-%, bevorzugt 5 - 10 Gew.-%, betragen.

Die Epoxyfettsäureester sind dabei entweder epoxidierte Fettsäureester einwertiger Alkohole, also z.B. epoxidierter Ölsäuremethylester, Linolsäuremethylester, Ricinolsäuremethylester oder epoxidierte Fettsäureester mehrwertiger Alkohole, z.B. Glycerinmonooleat oder Propylenglycol-monooleat oder epoxidierte Fettsäuretriglyceride, z.B. Ölsäuretriglycerid oder ungesättigte Öle wie z.B. Olivenöl, Sojaöl, Sonnenblumenöl, Leinöl, Rüböl.

Technisch besonders interessant sind vor allem ungesättigte Fettsäuremethylester-Epoxide aus ungesättigten Pflanzenfettsäuren. So ist als Esterpolyol das Umsetzungsprodukt eines Pflanzenölfettsäuremethylester-Epoxidats mit einem Polyol mit 2 - 6 C-Atomen und 2 - 6 Hydroxylgruppen besonders bevorzugt. Als Polyole können dabei z.B. Ethylenglycol, 1,2-Propylenglycol, 1,3-Propylenglycol, Butandiol, Pentandiol, Hexandiol, Glycerin, Trimethylolpropan, Pentaerythrit, Sorbit oder Diglycerin enthalten sein.

Besonders gut eignet sich dabei für die erfindungsgemäßen Haarbehandlungsmittel als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylester-Epoxid und Trimethylolpropan ist unter der Handelsbezeichnung Sovermol®760 erhältlich.

Ein weiterer Erfindungsgegenstand ist die Verwendung von Esterpolyolen, erhältlich durch Umsetzung von Epoxyfettsäureestern mit einem Epoxidsauerstoffgehalt von wenigstens 3 Gew.-% mit Wasser oder einem ein-oder mehrwertigen Alkohol mit 1 - 10 C-Atomen als lipidlösliche Wirkstoffe zur Spliß-Verminderung in Kombination mit wasserlöslichen haarsplißvermindernden Verbindungen in emulsionsförmigen Haarbehandlungsmitteln.

Als bekannte wasserlösliche haarsplißvermindernde Wirkstoffe können z.B. Zucker, also Mono- und Disaccharide enthalten sein. Geeignete Monosaccharide sind z.B. Glucose, Fructose, Mannose, aber auch Desoxyzucker wie z.B. Fucose oder Rhamnose. Geeignete Disaccharide sind z.B. Saccharose, Cellobiose, Lactose oder Maltose.

Ein weiterer haarsplißvermindernder, wasserlöslicher Wirkstoff ist das Nicotinsäureamid, das bevorzugt in einer Menge von 0,01 - 1 Gew.-% eingesetzt wird. Nicotinsäureamid, das auch als Vitamin B3 bezeichnet wird, ist als topisches Deodorans und auch als topischer Wirkstoff gegen Haarausfall beschrieben worden. Eine Wirkung auf die Verringerung von Haarspliß bei äußerlicher Anwendung auf das Haar ist bisher nicht beobachtet worden.

Weitere bekannte, im Wasser lösliche, haarsplißvermindernde Wirkstoffe sind das Panthenol und dessen Derivate, z.B. das kationisch derivatisierte Panthequat® (CTFA : Panthenyl-hydroxypropyl-steardimoniumchloride) sowie Polyvinylpyrrolidon. Diese wasserlöslichen Verbindungen werden bevorzugt als Kombination eingesetzt. Bevorzugt sind in den erfindungsgemäßen Haarbehandlungsmitteln 1 - 2 Gew.-% eines Zuckers und 0,1 - 1 Gew.-% Panthenol enthalten.

Ebenfalls bevorzugt ist in den erfindungsgemäßen Zubereitungen als wasserlöslicher Wirkstoff eine Kombination von Panthenol und Nicotinsäureamid enthalten. Das Gewichtsverhältnis dieser beiden wasserlöslichen Antispliß-Wirkstoffe kann zwischen 1 : 1 bis 10 : 1 liegen.

Die erfindungsgemäßen Haarbehandlungsmittel können darüber hinaus alle üblichen, für den jeweiligen Anwendungszweck gebräuchlichen Hilfsstoffe und Trägerkomponenten enthalten:

Dies sind zunächst die für emulsionsförmige Zubereitungen erforderlichen Öloder Fettkomponenten und Emulgatoren.

Als Öl- oder Fettkomponenten werden üblicherweise Paraffine bzw. Paraffinöle, natürliche oder synthetische Fettsäureester, Silikonöle, Fettalkohole oder Di-n-alkylether eingesetzt. Für Haarnachbehandlungsmittel sind z.B. Fettalkohole mit 14 - 22 C-Atomen, insbesondere Cetyl- und Stearylalkohol sehr bevorzugte Fettkomponenten. Aber auch Fettsäureester wie z.B. Isopropylmyristat, n-Hexyllaurat oder n-Decyloleat, synthetische Triglyceride wie z.B. Capryl-/Caprinsäure-Triglycerid oder natürliche Triglyceride wie z.B. raffinierte Pflanzenöle werden für solche Zwecke verwendet.

Zur Herstellung von Mikroemulsionen eignen sich als Ölkomponenten bevorzugt die Di-n-alkylether, z.B. der Di-n-octylether.

Als Emulgatoren können anionische, zwitterionische, kationische oder nichtionische oberflächenaktive Verbindungen eingesetzt werden. Die kationischen Tenside zeigen dabei den Vorteil, daß sie der statischen Aufladung des Haars entgegenwirken und auf diese Weise zur Verbesserung der Kämmbarkeit und zu einer Verringerung der Splißbildung beitragen.

Darüber hinaus ist bekannt, daß sich kationische Tenside zur Herstellung sehr niedrigviskoser Fettalkohol-Dispersionen eignen und für diesen Zweck bereits Mengen von 0,1 - 1 Gewichtsteilen kationischer, quartärer Ammoniumtenside ausreichend sind, um eine Dispersion von 10 - 25 Gewichtsteilen Cetyl- oder Stearylalkohol zu stabilisieren.

Bevorzugt werden als Ölkomponenten Gemische von Paraffinölen, Fettalkoholen und ggf. Fettsäureestern eingesetzt. Als Emulgatoren werden in solchen Fällen meist auch Gemische von kationischen und nichtionischen oberflächenaktiven Verbindungen eingesetzt. Als kationische oberflächenaktive Stoffe eignen sich z.B. Cetyl-trimethyl-ammoniumchlorid oder Di-stearoyloxyethyl-hydroxyethylmethyl-ammonium-methoxysulfat. Als nichtionische Emulgatoren können z.B. die Anlagerungsprodukte von 10 - 30 Mol Ethylenoxid an $C_{16}$ - $C_{22}$-Fettalkohole, an Fettsäuremonoglyceride, an Sorbitanfettsäuremonoester oder an Methylglucosid-fettsäuremonoester eingesetzt werden. Weitere geeignete nichtionische Emulgatoren sind z.B. Fettsäure-Polyglycerinester und deren Gemische mit Methylglucose-Fettsäureestern oder Alkyl-(oligo)-glycoside. Letztere werden bevorzugt zur Herstellung von Mikroemulsionen des Typs Öl-in-Wasser verwendet.

Als öllösliche Coemulgatoren werden darüber hinaus z.B. Fettsäuremono- oder -diglyceride, Sorbitanfettsäureester oder Propylenglycol-monofettsäureester verwendet.

Zur Viskositätseinstellung, z.B. zur Verdickung, können darüber hinaus die üblichen dafür bekannten wasserlöslichen Polymeren oder Cellulosederivate eingesetzt werden, wobei anionische Polymere wegen möglicher Interaktion nicht in Kombination mit kationischen Tensiden verwendet werden sollten. Bevorzugt eignen sich nichtionische polymere Verdickungsmittel wie z.B. Methylhydroxypropylcellulose, Hydroxypropylguar, Polyvinylpyrrolidon oder Hydroxyethylstärke.

Weitere Hilfsmittel, die in den erfindungsgemäßen Haarbehandlungsmitteln zur Erlangung spezieller haarkosmetischer Wirkungen enthalten sein können, sind z.B.

- Strukturanten wie z.B. Maleinsäure
- festigende Polymere
- haarkonditionierende Verbindungen, z.B. Phospholipide, z.B. Sojalecithin, Kephaline, Silikonöle, Proteine oder Proteinhydrolysate, kationische wasserlösliche Polymerisate, quaternisierte Proteinhydrolysate, Pflanzenextrakte, Allantoin, Pyrrolidoncarbonsäure-Salze, Vitamine
- Antischuppenwirkstoffe wie z.B. Zink Omadine, Piroctone Olamine, Salicylsäure und andere, gegen Pityrosporum ovale wirksame antimykotische Stoffe
- Lichtschutzmittel (UV-Licht absorbierende Stoffe)
- Haarfarbstoffe oder Farbstoffvorprodukte
- Oxidationsmittel zur Dauerwellfixierung, z.B. Alkalibromate, Wasserstoffperoxid.

Weiterhin können die üblichen Trägerstoffe und Formulierungshilfsmittel enthalten sein, die für die Stabilität, Viskosität, Aussehen, Geruch und Anwendungseignung erforderlich sind, z.B.

- Lösungsmittel und Lösungsvermittler, z.B. niedere Alkohole, Glycole, Glycerin, Diethylenglycol, Polyethylenglycol
- Farbstoffe, Trübungs- und Perlglanzmittel
- pH-Wert-Stellmittel und Puffer, z.B. Citronensäure-/citrat
- Komplexbildner, z.B. Acetophosphonate, EDTA oder NTA
- Konservierungsstoffe, z.B. Phenoxyethanol, p-Hydroxybenzoate
- Duftstoffe
- Antioxidantien und
- Aerosol-Treibgase

Die folgenden Beispiele sollen den Gegenstand der Erfindung näher erläutern:

**Beispiele**

Es wurden die folgenden Rezepturen hergestellt (Tabelle I) :

Tabelle I

| Zusammensetzung | 1 | 2 | V1 |
|---|---|---|---|
| Sovermol®760 (1) | 0,5 | 0,25 | - |
| Paraffinöl, dünnflüssig | 3,5 | 3,5 | 3,5 |
| Cetyl-/Stearylalkohol | 4,8 | 4,8 | 4,8 |
| Dehyquart®F 75 (2) | 0,35 | 0,35 | 0,35 |
| Cutina GMS (3) | 0,3 | 0,3 | 0,3 |
| Tego Care 450 (4) | 0,4 | 0,4 | 0,4 |
| PHB-Propylester | 0,2 | 0,2 | 0,2 |
| PHB-Methylester | 0,2 | 0,2 | 0,2 |
| Phenoxyethanol | - | 0,5 | 0,5 |
| Dehyquart A-CA (5) | 3,0 | 3,0 | 3,0 |
| Glucosemonohydrat | 2,0 | 2,0 | 2,0 |
| d-Panthenol | - | 0,25 | 0,5 |

Tabelle I   (fortgesetzt)

| Zusammensetzung | 1 | 2 | V1 |
|---|---|---|---|
| Nicotinsäureamid | - | 0,1 | - |
| Culminal MHPC 3000 (6) | 0,6 | 0,6 | 0,6 |
| Parfümöl | 0,25 | 0,25 | 0,25 |
| Wasser | ad 100 | ad 100 | ad 100 |
| pH-Wert | 3,5 | 4,25 | 3,7 |
| Viskosität [Pa·s], 20° C (Brookfield, Spindel 5, 4 Upm) | 54 | 53 | 30 |
| Spliß-Repair (%) R (korr.) | 20 | 41 | 14,3 |

Es wurden die folgenden Handelsprodukte verwendet:

| (1) | Sovermol®760 | Umsetzungsprodukt von Sojaölfettsäuremethylester-Epoxid mit 1 Mol Trimethylolpropan (pro Mol Epoxidsauerstoff) |
|---|---|---|
| (2) | Dehyquart®F 75 | Gemisch aus ca. 70 % Distearyloxyethylhydroxyethyl-methyl-ammonium-methoxysulfat und ca. 30 % Cetearyl-Alkohol |
| (3) | Cutina®GMS | Glycerin-(mono/di)-stearat/palmitat ca. 45 % Monoglycerid, ca. 40 % Diglycerid, ca. 10 % Triglycerid, ca. 2 % Glycerin |
| (4) | Tego Care 450 | Polyglycerin(3)/Methylglucose-Distearat |
| (5) | Dehyquart A-CA | Cetyl-trimethylammonium-chlorid |
| (6) | Culminal®MHPL 3000 | Methyl-Hydroxypropylcellulose (Viskosität 2 %ig in Wasser (20°C) : ca. 3 Pa.s) |

## Herstellung:

Die Komponenten der Ölphase (Sovermol 760, Paraffinöl, Cetyl-/Stearylalkohol, Dehyquart F75, Cutina GMS, Tego Care 450, PHB Ester und Phenoxyethanol wurden gemeinsam auf 80°C erhitzt. In die Schmelze wurden ca. 60 % des Wassers mit dem darin gelösten Dehyquart A-CA, ebenfalls auf 80°C erwärmt, unter Rühren einemulgiert. Zu der Emulsion wurde dann eine Lösung von Glucosemonohydrat, ggf. Panthenol und Nicotinsäureamid in ca. 20 % des Wassers sowie eine Quellung von Culminal MHPC in dem restlichen Wasser bei 40°C gegeben und unter Rühren eingemischt. Die Emulsion wurde weiter bis 30°C gerührt.
Der pH-Wert und die Viskosität wurden nach 20 Stunden Lagerung bei 20°C gemessen.

## Prüfung des Spliß-Repair-Effektes

Bündel von Haaren, die durch mechanische und elektrostatische Vorbehandlung (trockenes Kämmen) in hohem Maße gesplißt waren, wurden 15 Minuten lang mit den unverdünnten Zubereitungen gemäß Beispiel 1, 2 und V1 behandelt, dann mit Leitungswasser (16° d.H.) ausgespült und getrocknet. Die Splißrate wurde vor der Behandlung ($S_o$) und nach der Behandlung ($S_b$) durch Auszählen bestimmt und die Repair-Wirkung gemäß

$$R = \frac{S_o\text{-}S_b}{S_o} \cdot 100\ [\%]$$

ermittelt.

Da reines Wasser eine Repair-Wirkung nach dieser Methode von R = 30 % aufweist, wurde die Spliß-Repair-Wirkung der Versuchsprodukte wie folgt korrigiert

$$R_{(korr)} = \frac{R\text{-}30}{70} \cdot 100\ [\%]$$

Die erhaltenen Spliß-Repair-Werte $R_{(korr)}$ sind in der Tabelle I angegeben.

**Patentansprüche**

1. Zubereitung zur Behandlung der Haare in Form einer Öl-in-Wasser-Emulsion, **dadurch gekennzeichnet, daß** als splißvermindernde Wirkstoffe

0,1 - 10 Gew.-% eines lipidlöslichen Esteralkohols oder Esterpolyols, erhältlich durch Umsetzung eines Epoxyfettsäureesters mit Wasser oder einem ein- oder mehrwertigen Alkohol mit 1 - 10 C-Atomen als Komponente der Ölphase und

0,1 - 3 Gew.-% einer bekannten haarsplißvermindernden wasserlöslichen Verbindung aus der Gruppe Panthenol, Nicotinsäureamid, Zucker, Polyvinylpyrrolidon oder Gemische davon als Komponente der wäßrigen Phase

enthalten sind.

2. Zubereitung nach Anspruch 1 , **dadurch gekennzeichnet, daß** als Esterpolyol das Umsetzungsprodukt eines Pflanzenölfettsäuremethylester-Epoxidats mit einem Polyol mit 2 - 6 C-Atomen und 2 - 6 Hydroxylgruppen enthalten ist.

3. Zubereitung nach einem der Ansprüche 1 oder 2, **dadurch gekennzeichnet, daß** als Esterpolyol das Umsetzungsprodukt eines Pflanzenölfettsäuremethylester-Epoxidats mit Trimethylolpropan mit einer Hydroxylzahl von 350 - 450 enthalten ist.

4. Zubereitung nach einem der Ansprüche 1 - 3, **dadurch gekennzeichnet, daß** als wasserlösliche Wirkstoffe Panthenol und Nicotinsäureamid enthalten sind.

**Claims**

1. A hair treatment preparation in the form of an oil-in-water emulsion, **characterized in that** it contains 0.1 to 10% by weight of a lipid-soluble ester alcohol or ester polyol obtainable by reacting an epoxyfatty acid ester with water or a mono- or polyhydric alcohol containing 1 to 10 carbon atoms as a component of the oil phase and 0.1 to 3% by weight of a known, split-end-reducing water-soluble compound from the group consisting of panthenol, nicotinic acid amide, sugar, polyvinyl pyrrolidone or mixtures thereof as a component of the aqueous phase as ingredients active in reducing split ends.

2. A preparation as claimed in claim 1, **characterized in that** it contains the reaction product of a vegetable oil fatty acid methyl ester epoxidate with a polyol containing 2 to 6 carbon atoms and 2 to 6 hydroxyl groups as the ester polyol.

3. A preparation as claimed in claim 1 or 2, **characterized in that** it contains the reaction product of a vegetable oil methyl ester epoxidate with trimethylol propane having a hydroxyl value of 350 to 450 as the ester polyol.

4. A preparation as claimed in any of claims 1 to 3, **characterized in that** it contains panthenol and nicotinic acid amide as water-soluble active ingredients.

**Revendications**

1. Préparation pour le traitement de cheveux sous forme d'une émulsion huile-dans-eau, **caractérisée en ce qu'**elle contient comme substances actives diminuant les fourches 0,1 à 10% en poids d'un ester-alcool ou d'un ester-polyol soluble dans les lipides, pouvant être obtenu par transformation d'un ester d'acide gras époxy avec de l'eau ou un alcool monovalent ou polyvalent comprenant 1 à 10 atomes de carbone comme composant de la phase huileuse et 0,1 à 3% en poids d'un composé connu soluble dans l'eau, diminuant les fourches des cheveux, du groupe constitué par le panthénol, l'amide de l'acide nicotinique, le sucre, la polyvinylpyrrolidone ou les mélanges de ceux-ci comme composant de la phase aqueuse.

2. Préparation selon la revendication 1, **caractérisée en ce qu'**elle contient comme ester-polyol le produit de transformation d'un époxydat d'ester méthylique d'un acide gras d'huile végétale avec un polyol de 2 à 6 atomes de carbone et 2 à 6 groupes hydroxyle.

3.  Préparation selon l'une quelconque des revendications 1 ou 2, **caractérisée en ce qu'**elle contient comme ester-polyol le produit de transformation d'un époxydat d'ester méthylique d'acide gras d'huile végétale avec du triméthylolpropane avec un indice d'hydroxyle de 350 à 450.

4.  Préparation selon l'une quelconque des revendications 1 à 3, **caractérisée en ce qu'**elle contient comme substances actives solubles dans l'eau du panthénol et de l'amide de l'acide nicotinique.